# EUROPEAN PATENT SPECIFICATION

(11) **EP 2 323 613 B1**
(45) Date of publication and mention of the grant of the patent: **30.11.2016**
(21) Application number: 09807075.8
(22) Date of filing: 04.08.2009
(51) Int. Cl.: A61K 8/11, A61K 9/48, A61K 8/29, A61K 8/27, A61K 8/34, A61Q 17/04, A61P 17/18

(54) **COMPOSITE PARTICLES HAVING AN ANTIOXIDANT-BASED PROTECTIVE SYSTEM, AND TOPICAL COMPOSITIONS COMPRISING THE SAME**
KOMPOSITPARTIKEL ENTHALTEND EIN ANTIOXIDANT-BASIERENDES SCHUTZSYSTEM, SOWIE TOPISCHE ZUSAMMENSETZUNGEN DARAUS
PARTICULES COMPOSITES AYANT UN SYSTÈME PROTECTEUR À BASE D'ANTIOXYDANT ET COMPOSITIONS TOPIQUES LES COMPRENANT

(30) Priority: 12.08.2008 US 88032 P
(43) Date of publication of application: 25.05.2011
(73) Proprietor: ELC Management LLC, New York, NY 10153 (US)
(72) Inventor: SENTE, Ilse, B-3520 Zonhoven (BE); DECLERCQ, Lieve, B-2180 Ekeren (BE); MAES, Daniel H., Huntington New York 11743 (US); SOJKA, Milan Franz, Coram New York 11727 (US); CUMMINS, Phillip, Livingston New Jersey 07039 (US); FTHENAKIS, Christina G., Dix Hills New York 11746 (US); PERNODET, Nadine A., Huntington NewYork 11743 (US); LEE, (Dr.) Wilson A., Happauge New York 11788 (US); NAJDEK, Linda, East Islip New York 11730 (US); McKEEVER-ALFIERI, MaryAnn, East Islip New York 11730 (US); TETA, Lawrence P., Centereach New York 11720 (US)
(74) Representative: Hirsch & Associés
(86) International application number: PCT/US2009/052683
(87) International publication number: WO 2010/019413

(56) References cited:
- WO-A2-2009/079135
- US-A1- 2005 112 154
- US-A1- 2005 208 005
- US-A1- 2007 071 978
- US-B1- 6 814 959
- LEE WILSON A ET AL: "Multicomponent polymer coating to block photocatalytic activity of TiO2 nanoparticles.", 7 December 2007 (2007-12-07), CHEMICAL COMMUNICATIONS (CAMBRIDGE, ENGLAND) 7 DEC 2007, NR. 45, PAGE(S) 4815 - 4817, XP002725104, ISSN: 1359-7345 * the whole document *

## Description

### CROSS-REFERENCE TO RELATED APPLICATIONS

This claims priority to U.S. Provisional Patent Application No. 61/088,032 filed August 12, 2008.

### FIELD OF THE INVENTION

The present invention relates to topical compositions comprising composite particles with a unique antioxidant-based protective system for enhanced protection of the skin against oxidative stress caused by exposure to ultra-violet (UV) light or other environmental factors.

### BACKGROUND OF THE INVENTION

Reactive oxygen species (ROS), such as oxygen ions, free radicals, and peroxides (either inorganic or organic), are natural byproducts of the normal metabolism of oxygen by living cells. On one hand, ROS play important roles in cell signaling, a process termed redox signaling, and they are also used by the immune system to attack and kill pathogens, thereby protecting the living cells against invasion by such pathogens. On the other hand, reactive oxygen species, if not reduced or eliminated timely, may cause extensive damage to all components of living cells, including proteins, lipids, and DNA. Thus, to maintain proper cellular homeostasis, a balance must be struck between the production and consumption of ROS. Various enzymes produced by the living cells, such as superoxide dismutase, catalase, and glutathione peroxidase, function as cellular antioxidants to eliminate the excess reactive oxygen species. Consequently, the reactive oxygen species are present only at low levels in normal living cells, and the damage caused by them is constantly repaired by various cellular repair mechanisms. However, during times of environmental stress, the ROS levels can increase dramatically, which may lead to an imbalance between the production of ROS by a biological system and the biological system's capability to detoxify the reactive intermediates or repair the resulting damages. This cumulates into a situation commonly referred to as "oxidative stress." Oxidative stress is involved in many diseases, such as atherosclerosis, Parkinson's disease and Alzheimer's disease. Oxidative stress is also believed to be a major contributor to the aging process.

Lee Wilson et al., Multicomponent polymer coating to block photocatalytic activity of TiO₂ nanoparticules; Chemical Communications, 45, 4815-17 (2007) describes polymer coating used to block photocatalytic activity of TiO₂ nanoparticles. Grafting of anti-oxidant molecules is used, and grafting an additional hydrophobic polymer coating is said to stabilize the anti-oxidant.

US 6,814,959 relates to UV radiation reflecting or absorbing agents, designed to be applied on the skin, the mucous membranes, the scalp and the hair for protection against harmful UV radiation and to reinforce the natural skin barrier. The described agents comprise polymorphous, crystalline or semi-crystalline solid polymeric or lipidic particles. additional substances may be added; such as UV blockers, anti-oxidatives substances ...

The skin is an organ where ROS are particularly liable to be formed, because it is directly exposed to various environmental assaults. Over the course of time under the cumulative influence of oxidative stress, the skin will start to show various signs of aging, such as thinning of the stratum corneum layer, loss of firmness and tonicity, excessive dryness, and appearance of fine lines and wrinkles.

It is therefore desirable to provide a topical composition with strong antioxidant activities to scavenge excess ROS and to protect skin against the harmful effects of oxidative stress.

Certain particles commonly used in cosmetic compositions, such as iron oxides, titanium dioxide, and zinc oxide, are known to cause generation of ROS, which not only can cause oxidative stress on the skin, but also may interfere with other ingredients or components in the cosmetic compositions. For example, many organic dyes/colorants, organic sunscreen agents, and other organic cosmetic ingredients are known to be susceptible to oxidative decomposition or degradation. Combined use of the ROS-re leasing particles with such organic cosmetic ingredients may consequently lead to in situ decomposition or degradation of such organic cosmetic ingredients and adversely affect the overall performance and stability of the cosmetic compositions.

There is therefore a continuing need for treating or modifying the ROS-releasing particles to eliminate or reduce any potential oxidative stress that such particles may exert on the skin and to prevent such particles from causing the decomposition or degradation of other cosmetic ingredients.

### SUMMARY OF THE INVENTION

The present invention provides composite particles containing one or more core particles capable of causing generation of reactive oxygen species (ROS), either alone or upon exposure to ultra-violet (UV) light or other environmental factors, while the core particles are encapsulated within a polymeric shell with a unique antioxidant-based protective system. Such antioxidant-based protective system functions to eliminate or reduce any potential oxidative stress that the ROS-releasing core particles may exert on the skin and to prevent such core particles from causing the decomposition or degradation of other cosmetic ingredients, and it is also capable of scavenging excess ROS in the surrounding environment and thereby protecting skin against any potential harmful effects of oxidative stress.

In one aspect, the present invention relates to a topical composition containing a dispersion of composite particles in a cosmetically or pharmaceutically acceptable medium, wherein each of the composite particles has one or more core particles encapsulated within a polymeric shell, wherein at least some of the core particles contains a material capable of causing generation of ROS, wherein a first antioxidant capable of quenching or scavenging ROS is co-encapsulated or co-entrapped with the core particles inside the polymeric shell, and wherein a second antioxidant capable of preventing or reducing oxidative damage to the skin is coated over the polymeric shell.

In another aspect, the present invention relates to a composite particle containing one or more core particles encapsulated within a polymeric shell, wherein at least some of the core particles contains a material capable of causing generation of ROS, wherein a first antioxidant capable of quenching or scavenging ROS is co-encapsulated or co-entrapped with the core particles inside the polymeric shell, and wherein a second antioxidant capable of preventing or reducing oxidative damage to the skin is coated over the polymeric shell.

Other aspects and objectives of the present invention will become more apparent from the ensuing description, examples, and claims.

### BRIEF DESCRIPTION OF THE DRAWINGS

FIG. 1 is a schematic cross-sectional view of an exemplary composite particle, according to one embodiment of the present invention.
FIG. 2 is a schematic cross-sectional view of another exemplary composite particle, according to an alternative embodiment of the present invention.

### DETAILED DESCRIPTION OF THE INVENTION,AND

### PREFERRED EMBODIMENTS THEREOF

The composite particles of the present invention, by utilizing a unique protective system containing two different antioxidants placed at two different locations, are capable of significantly reducing or completely eliminating generation of reactive oxygen species (ROS) caused by the core particles while simultaneously providing improved protection of the skin against oxidative damage.

Any inorganic or organic solid particles that are capable of causing generation of ROS can be used as the core particles to form the composite particles of the present invention. For example, the core particles can be formed of certain metal oxides, such as iron oxides, zinc oxide and titanium dioxide. More specifically, the core particles of the present invention may comprise either titanium dioxide or zinc oxide. Zinc oxide and titanium dioxide particles are known to have photoprotective characteristics and are particularly desirable components in sunscreen compositions. However, zinc oxide and titanium dioxide particles in their "naked" or untreated states are also known to cause generation of ROS upon exposure to ultra-violet (UV) light and consequently lead to oxidative stress on the skin as well as oxidative decomposition or degradation of other organic cosmetic components in the surrounding environment, such as, for example, organic dyes or organic sunscreen agents. Therefore, it is important to treat or modify zinc oxide and titanium dioxide particles so as to overcome the above-described drawbacks. In a particularly preferred embodiment of the present invention, some of the core particles in the composition particles of the present invention comprise titanium dioxide, while others comprise zinc oxide. The core particles as used in the present invention can also be formed of organic cosmetic ingredients that are capable of generating or causing generation of ROS, such as unsaturated lipids, organic dyes or colorants, organic sunscreen agents, fragrance compounds, essential oils, and the like.

The core particles used in the present invention can be of any regular or irregular shape, such as spherical, cubic, cylindrical, planar, fibrous, and the like. The average particle size of the core particles as used herein may range from about 0.001 micron to 75 microns. Preferably, but not necessarily, the particles are less than about 1 micron, more preferably ranging from about 0.001 micron to about 0.1 micron, and most preferably from about 0.01 micron to about 0.05 micron.

As described hereinabove, the core particles of the present invention are encapsulated or entrapped inside a polymeric shell. The polymeric shell of the present invention may comprise any suitable synthetic or natural polymer. Preferably, but not necessarily, the polymeric shell as used in the present invention comprise at least one synthetic polymer obtained by polymerization of one or more ethylenically unsaturated monomers to form homopolymers or copolymers of ethylenically unsaturated monomers, or copolymers of ethylenically unsaturated monomers and one or more organic groups. Examples of ethylenically unsaturated monomers that may be suitable for the practice of the present invention include, for example, vinylidene chloride, vinyl chloride, acrylonitrile, acrylic acid and its corresponding C1-C20 aliphatic or aromatic esters, methacrylic acid and its corresponding C1-C20 aliphatic or aromatic esters, acrylamide, methacrylamide, vinyl pyrrolidone, alkenes such as styrene, ethylene, propylene, butylene, methylpentene, 1,3-butadiene, and the like. The polymeric shell of the present invention is formed of suitable synthetic polymers selected from polyesters, polyamides, polyphthalamides, polyimides, polycarbonates, polyketones, cellulose acetate, polysulfones, polyphenylene sulfides, polyphenylene oxides, polylactic acids, polyvinylpyrrolidone, polystyrene, polyacrylonitrile, polyacrylamide, polymethylmethacrylate, polyacrylates, and copolymers of the above-listed polymers.

In a particularly preferred embodiment of the present invention, the core particles are entrapped within a collapsed polymeric shell, which is formed from a hollow microsphere with a deformable polymeric shell that encapsulates therein an expandable fluid. Specifically, such hollow microspheres are mixed with the core particles to be entrapped and a polar organic solvent. The polar organic solvent causes the deformable polymeric shells of the hollow microspheres to swell, but without dissolving the same, so that multiple microchannels are formed in the swelled polymeric shells to allow entry of the core particles into the hollow microspheres and exit of the expandable fluid therefrom. Correspondingly formed are microspheres that each comprises a collapsed polymeric shell with one or more of the core particles entrapped therein. The entrapment process and the materials used in forming such microspheres are described in greater detail in co-pending U.S. Patent Application No. 12/138,742 filed on June 13, 2008 for "COMPOSITIONS COMPRISING SOLID PARTICLES ENTRAPPED IN COLLAPSED POLYMERIC MICROSPHERES, AND METHODS OF MAKING THE SAME," the content of which is incorporated herein by reference in its entirety for all purposes.

In order to effectively abate or eliminate reactive oxygen species (ROS) released by the core particles, a first antioxidant, which is a ROS-scavenger, is co-encapsulated or co-entrapped with the core particles inside the polymer shell. In this manner, the first antioxidant becomes localized or immobilized in close proximity to the core particles and is therefore capable of quenching or scavenging any ROS generated in the vicinity of the core particles with significantly improved effectiveness.

Any suitable ROS-scavenger can be used as the first antioxidant of the present invention, which includes, but is not limited to: singlet-oxygen scavenger, superoxide scavenger, hydroxyl radical scavenger, or mixture or combination thereof. For example, the first antioxidant may be selected from the following broad categories: (A) tocopherols (such as vitamin E), tocotrienols and their derivatives such as acetates and succinates; (B) carotenoids such as alpha-carotene, beta-carotene (also known as Vitamin A), gamma-carotene, lycopene, lutein, beta-crytoxanthin, zeaxanthin and astaxanthin; (C) minerals such as zinc, selenium and magnesium, while selenium-containing compounds further include selenoproteins; (D) polyphenols such as flavonoids, phenolic acids, and non-acid phenolic compounds such as ellagitannis, gallotannis and condensed tannins; (E) lipoic acids such as alpha-lipoic acid and dihydrolipoic acid; (F) transition metal ion-binding proteins such as ceruloplasmin, lactoferrin and transferrin; (G) melatonin; (H) hormones and hormone-related compounds, such as estrogens, thyroxine, and dehydroepiandrosterone; (I) polyamines such as cadaverine, putrescine, spermidine and spermine; (J) tamoxifen and its metabolites such as 4-hydroxytamoxifen; (K) propofol; (L) cinnamic acids and derivatives thereof; (M) coumarins; (N) stilbenes, such as resveratrol; (O) proteins such as albumin and amino acids such as creatine. Other antioxidants that can also be used as the first antioxidant in the composite particles of the invention include terpenoids, organosulfur compounds, indoles, lignans, coenzyme Q, uric acid, copper, and pycnogenol.

More specifically, the first antioxidant of the present invention can be selected from the group consisting of *Acacia catechu* bark extract, alpha-tocopherol, anthocyanins, *Argania spinosa* leaf extract, aronia (chokeberry) extract, ascorbic acid and esters thereof, *Aspalathus linearis* (rooibos) extract, baicalin, bakuchiol, bamboo extract, bamboo leaf extract, benfotiamine, butylated hydroxytoluene (BHT), *Camellia sinensis* extract, catechins, chlorogenic acids, *Chondrus crispus* (carrageenan) extract, citrus peel extract, cocoa extract, cocoa polyphenols, cranberry extract, decarboxy carnosine HCl, diethylhexyl syringylidenel maloneate, epigallicathecin gallate (EGCG), ethylbisiminomethylguaiacol manganese chloride (EUK-134), ferulic acid, ferulic acid monophosphate, *Ficus carica* (common fig) extract, gamma-oryzanol, grape seed extract, grape skin extract, 4-hydroxy-isoleucine extract, 6-hydroxy-2,5,7,8-tetramethylchroman-2-carboxylic acid and phosphate thereof, hydroxy dimethoxybenzyl malonate, *Hypoxis hemerocallidea* (African potato) extract, idebenone, kaempferol, kiwi seed extract, kola nut (cola) powder, L-ergothioneine, L-histidine, lipoic acid and esters thereof, litchi seed extract, L-2-oxo-thiazolidine (OTZ), lutein, luteolin, manganese gluconate, mannitol, *Morinda citrifolia* (noni) leaf extract, myricetin, N-acetyl cysteine (NAC), pentagalloylglucose (PGG), *Phyllanthus emblica* (Indian gooseberry) extract, quercetin, quercetin hydrate, quercitrin, red wine extract, resveratrol and derivatives thereof, *Ribes nigrum* (blackcurrent) extract, *Rhodiola rosea* (golden root) extract, *Rosa roxburghii* (chestnut rose) fruit extract, rosemary extract, rosemarinic acid, rutin, salicylic acid, salicyloyl cyclic phosphate, scutellaria extract, *Scutellaria baicaleszsis* root extract, *Siringa vulgaris* (lilac) extract, spinach extract, tetrahydrocurcuminoids, tocopherol and esters thereof, vanilla extract, walnut polyphenols, and mixtures or combinations thereof. Among the above-listed antioxidants, N-acetyl cysteine (NAC), gamma-oryzanol, and tetrahydrocurcuminoids are particularly preferred as the first antioxidant, while gamma-oryzanol is the most preferred.

Although the ROS generated in the vicinity of the core particles can be completely eliminated or significantly reduced by the first antioxidant inside the polymeric shell of the composite particles of the present invention, the human skin may still be subject to oxidative stress caused by other environmental factors, such as ultra-violet (UV) light, smoke, harsh chemicals, and the like, which can lead to oxidative damage to lipids, proteins and DNA in the skin cells. Lipids are usually the first target of such oxidative assaults, and the level of lipid peroxidation is therefore commonly used as a bio-marker indicative of the oxidative damage to the skin. The term "lipid peroxidation" refers to the oxidative degradation of either extracellular lipids or lipids in cell membranes, which results in cell damage. This process may for example proceed through a free radical chain reaction, in which ROS first reacts with a hydrogen atom to form a fatty acid radical and water, the fatty acid radical (which is not stable by itself) then readily reacts with molecular oxygen to form a peroxyl-fatty acid radical, the peroxyl-fatty acid radical (which is also not stable by itself) subsequently reacts with another free fatty acid to form a different fatty acid radical and a hydrogen peroxide or reacts with itself to form a cyclic peroxide. The chain reaction will proceed with a new fatty acid radical formed at each cycle, until the fatty acid radicals react with each other to produce non-radical species or are eliminated by antioxidants in the surrounding environment. If this chain reaction is not terminated fast enough, it can lead to significant damage to the cell membrane. A second pathway is through singlet oxygen generation by an endogenous photosensitizer, which can initiate the formation of lipid peroxides. Further, the oxidation end products may be mutagenic or carcinogenic. For example, the end product malondialdehyde can react with DNA and thereby lead to DNA damage and mutation.

In order to protect the skin against the above-described deleterious effects of the above-described oxidative damage to the skin, a second antioxidant capable of preventing or reducing oxidative damage to the skin, including skin lipid peroxidation, is provided. Such second antioxidant overcoats the polymeric shell of the composite particle of the present invention, so that it can be released into the surrounding environment over time to combat oxidative stress and protect the skin against potential oxidative damage. Any suitable antioxidants capable of preventing or reducing oxidative damage to the skin, including skin lipid peroxidation, can be used as the second antioxidant in the present invention, which includes, but is not limited to: ascorbyl palmitate, ascorbyl stearate, ascorbyl tocopheryl maleate, butylated hydroxyanisole (BHA), butylated hydroxytoluene (BHT), *Caesalpinia paraensis* extract, chlorogenic acids, *Polypodium leucotomos* extract, *Acacia catechu* bark extract, alpha-tocopherol, anthocyanins, , *Argania spinosa* leaf extract, aronia (chokeberry) extract, ascorbic acid and esters thereof, ascorbyl tocopheryl maleate (2-CME), *Aspalathus linearis* (rooibos) extract, baicalin, bakuchiol, bamboo extract, bamboo leaf extract, benfotiamine, butylated hydroxytoluene (BHT), *Camellia sinensis* extract, *Chamomilla recutita* (matricaria) flower oil, catechins, chlorogenic acids, *Chondrus crispus* (carrageenan) extract, citrus peel extract, cocoa extract, cocoa polyphenols, cranberry extract, decarboxy carnosine HCl, diethylhexyl syringal malonate, epigallicathecin gallate (EGCG), ethylbisiminomethylguaiacol manganese chloride, ferulic acid, ferulic acid monophosphate, Ficus carica (common fig) extract, gamma-oryzanol, Garcinia mangostana peel extract, glucosylrutin, grape seed extract, grape skin extract, 4-hydroxy-isoleucine extract, 6-hydroxy-2,5,7,8-tetramethylchroman-2-carboxylic acid and phosphate thereof, hydroxy dimethoxybenzyl malonate, Hypoxis hemerocallidea (African potato) extract, idebenone, kaempferol, kiwi seed extract, kola nut (cola) powder, L-ergothioneine, L-histidine, Helianthus annuus (sunflower) seed extract, licorice extract, lipoic acid and esters thereof, litchi seed extract, L-2-oxo-thiazolidine (OTZ), lutein, luteolin, manganese gluconate, mannitol, Mimosa tenuiflora bark extract, Morinda citrifolia (noni) leaf extract, nordihydroguaiaretic acid (NDGA), myricetin, N-acetyl cysteine (NAC), Nymphea alba (water lily) flower extract, Oenothera biennis (evening primrose) seed extract, Oryza sativa (rice) extract, pentagalloylglucose (PGG), Perilla ocymoides seed extract, {Phyllanthus emblica (Indian gooseberry) extract, Pimpinella anisum (anise) fruit extract, Pinus pinaster (French maritime pine) bark extract, Psoralea corylifolia seed extract, Punica granatum (pomegranate) extract quercetin, quercetin hydrate, quercitrin, red wine extract, resveratrol and derivatives thereof, Ribes nigrum (blackcurrent) extract, Rhodiola rosea (golden root) extract, Rosa roxburghii (chestnut rose) fruit extract, rosemary extract, rosemarinic acid, rutin, rutin hydrate, salicylic acid, salicyloyl cyclic phosphate, sodium azide, Scutellaria extract, Scutellaria baicaleszsis root extract, Siringa vulgaris (Lilac) extract, spinach extract, tetrahydrocurcuminoids, Thermus thermophillus ferment, tocopherol and esters thereof, tocotrienols, ubiquinone, Vaccinium myrtillis (bilberry) extract, vanilla extract, walnut polyphenols, wasabi extract, xanthophyll, and mixtures or combinations thereof. Among the above-listed antioxidants, ascorbyl tocopheryl maleate, which is commercially available under the trademark 2-CME™ from Senju Pharmaceutical Co., Ltd. in Japan, is particularly preferred as the second antioxidant.

The above-described second antioxidant is preferably attached to the polymeric shell by either chemical or physical bonds. For example, the second antioxidant can be linked to the polymeric shell by covalent bonds, hydrogen bonds, or Van der Waals force. Regardless of the mechanism of attachment, it is preferred that the second antioxidant can be readily detached from the composite particles and released into the surrounding environment after application
onto the skin, so as to protect the skin against potential oxidative damage such as lipid peroxidation.

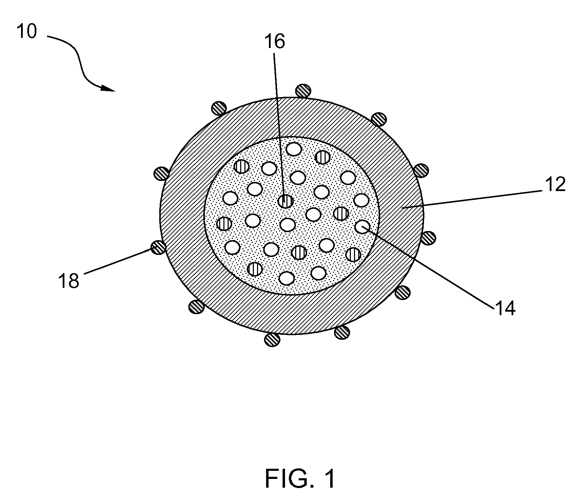

FIG. 1 shows a schematic cross-sectional view of an exemplary composite particle, according to one embodiment of the present invention. Specifically, the composition particle 10 comprises a polymeric shell 12, which encapsulates or entraps therein core particles 14, which are capable of releasing ROS, and a first antioxidant 16, which is capable of scavenging or reducing the ROS released by the core particles 14. A second antioxidant 18 capable of preventing or reducing skin lipid peroxidation is coated over the polymeric shell 12. Preferably, the second antioxidant 18 is attached to the outer surface of the polymeric shell 12 by covalent bonds, hydrogen bonds, or Van de Waals force.

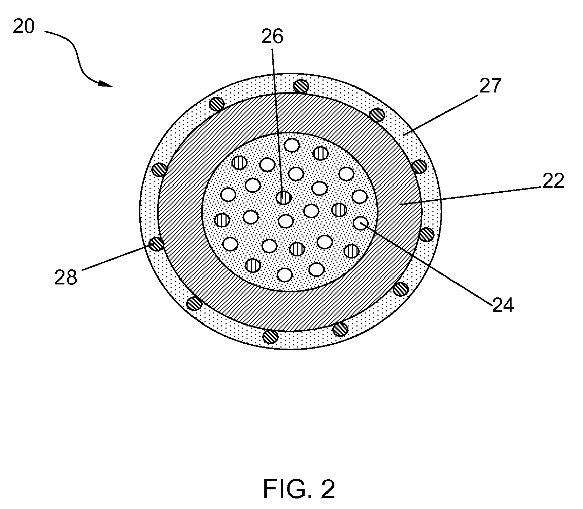

FIG. 2 shows a schematic cross-sectional view of another exemplary composite particle, according to an alternative embodiment of the present invention. Specifically, the composition particle 20 comprises a polymeric shell 22, which encapsulates or entraps therein core particles 24, which are capable of releasing ROS, and a first antioxidant 26, which is capable of scavenging or reducing the ROS released by the core particles 24. A second polymeric layer 27, which contains a second antioxidant 28 capable of preventing or reducing skin lipid peroxidation, is coated over the polymeric shell 22. The second polymeric layer 27 can be formed of any suitable polymeric material, which can be either the same or different from the polymeric material that forms the shell 22. Suitable materials for forming the second polymeric layer 27 include film-forming materials such as natural or synthetic homo- or copolymers comprised of ethylenically unsaturated monomers including acrylic acid, methacrylic acid or their C₁-C₁₀ alkyl esters, ethylene, propylene, or vinylpyrrolidones ; silicone gums, which are organosiloxanes generally having a viscosity ranging from about 200,000 to 10,000,000 centipoise at room temperature; animal, vegetable, silicone or mineral waxes; organic ester or hydrocarbon oils, or silicone resins such as trimethylsiloxy silicate or polymethylsilsesquioxane; cellulosic polymers; fatty acids (e.g. fatty carboxylic acids having from about 6 to 40 carbon atoms that may be liquid, solid or semi-solids at room temperature), fatty alcohols (e.g. alcohols having from 6 to 50 carbon atoms that may be liquid, solid, or semi-solid at room temperature), and inorganic materials. Preferably, but not necessarily, the film-forming material comprises an alkyl silicone polymer or more specifically a fatty alkylmethylsiloxane, such as cetyl dimethicone, stearyl dimethicone, or behenyl dimethicone, or other modified siloxanes, such as polyoxyalkylenated silicones typically referred to as dimethicone copolyol or cetyl dimethicone copolyol. For example, a polymethylhydrogensiloxane, which is commercially available from Dow Corning Corporation at Midland, MI under the trade name of Dow Corning^{®} MH 1107 fluid, can be used as the film-forming material in the present invention. This polymethylhydrogensiloxane material is a colorless silicone liquid that can be heat cured in the presence of a catalyst (e.g., zinc octoate, iron octoate, dibutyl tin dilaurate, and tin octoate) to form a solid, liquid-impermeable membrane comprised of cross-linked dimethicone over the microspheres of the present invention. For another example, silicone copolymers commercialized by Dow Corning under the trade name of BIO-PSA, which are formed by reacting a siloxane resin with a diorganosiloxane, can also be used as film-forming materials in the present invention to form the second polymeric layer 27 over the polymeric shell 22. Among various types of BIO-PSA materials available from Dow Corning, the Dow Corning^{®} 7-4404, 7-4405, and 7-4411 fluids (containing trimethylated silica treated with dimethylsiloxane and dispersed in a cosmetically acceptable solvent, such as octamethyltrisiloxane, isododecane, or decamethyltetrasiloxane) are particularly preferred.

The composite particles of the present invention as described hereinabove may have an average particle size ranging from about 1 to about 100 microns, more preferably from about 1 to 50 microns, even more preferably from about 1 to about 15 microns, and most preferably from about 5 to about 8 microns, as determined by a Malvern Particle Size Analyzer, available from Malvern Instrument at Worcestershire, UK. The core particles may account for from about 5 to about 90% of the total weight of the resulting composite particles, more preferably 10% to about 75% and most preferably from about 30% to about 60% of the total weight. The polymeric shell may account for from about 5% to about 75% of the total weight of the resulting composite particles, more preferably from about 10% to about 60% and most preferably from about 30% to about 50% of the total weight. The first antioxidant may account for from about 0.1 % to about 30% of the total weight of the resulting composite particles, more preferably from about 0.2% to about 15% and most preferably from about 1% to about 5% of the total weight. The second antioxidant may account for from about 0.1% to about 30% of the total weight of the resulting composite particles, more preferably from about 0.2% to about 15% and most preferably from about 1% to about 5% of the total weight. The optional second polymeric layer as described hereinabove in FIG. 2 may account for from about 1% to about 30% of the total weight of the resulting composite particles, more preferably from about 5% to about 20% and most preferably from about 10% to about 15% of the total weight.

The composite particles of the present invention can be added directly to any pharmaceutically or cosmetically acceptable carrier to form a cosmetic or topical composition. For purpose of the present invention, pharmaceutically or cosmetically acceptable carriers are substances that are biologically compatible with human skin and can be used to formulate active ingredients described hereinabove and/or hereinafter into a cream, gel, emulsion, liquid, suspension, powder, nail coating, skin oil, or lotion that can be topically applied. In the case where the cosmetically acceptable carrier is in the form of an emulsion, it may contain from about 0.1 to 99%, preferably from about 0.5 to 95%, more preferably from about 1 to 80% by weight of the total composition of water and from about 0.1 to 99%, preferably from about 0.1 to 80%, more preferably from about 0.5 to 75% by weight of the total composition of oil. In the case where the composition is anhydrous it may comprise from about 0.1 to 90 wt% of oil and from about 0.1 to 75 wt% of other ingredients such as pigments, powders, non-aqueous solvents (such as mono-, di-, or polyhydric alcohols, etc. In the case where the composition is in the form of an aqueous based gel, solution, or suspension, it may comprise from about 0.1 to 99 wt% of water and from about 0.1 to 75 wt% of other ingredients such as botanicals, non-aqueous solvents, etc.

The pharmaceutically or cosmetically acceptable carrier or carriers can be present in the topical or cosmetic composition of the present invention at an amount ranging from about 0.1% to about 99.9%, preferably from about 5% to about 99.5%, more preferably from about 10% to about 99%, and most preferably from about 10% to 90% by total weight of the topical or cosmetic composition.

The topical or cosmetic composition may contain one or more skin care actives, which are agents that provide benefits to the skin, rather than merely improving the physical or aesthetic characteristics of the topical composition. If present, such skin care actives may range from about 0.01 to 50%, preferably from about 0.05 to 35% by weight of the total composition. Exemplary skin care additives that can be used in the topical or cosmetic compositions of the present invention include, but are not limited to: chemical or physical sunscreens, self-tanning agents such as dihydroxyacetone, anti-acne agents (e.g., resorcinol, salicylic acid, benzoyl peroxide, and the like), enzyme-inhibiting agents, collagen-stimulating agents, agents for the eradication of age spots and keratoses, analgesics, anesthetics, antimicrobials (e.g., antibacterials, antiyeast agents, antifungal agents, and antiviral agents), antidandruff agents, antidermatitis agents, antipruritic agents, antiemetics, anti-inflammatory agents, antihyperkeratolytic agents, antiperspirants, antipsoriatic agents, antiseborrheic agents, antihistamine agents, skin lightening agents, depigmenting agents, skin soothing/healing agents (e.g., aloe vera extract, allantoin, and the like), corticosteroids, hormones, proteins or peptides, vitamins and derivatives thereof (e.g., vitamin A, vitamin E, vitamin B₃, vitamin B₅, and the like), exfoliants, retinoids (e.g., retinoic acid and retinol), farnesol, bisabolol, phytantriol, glycerol, urea, guanidine (e.g., amino guanidine), clotrimazole, ketoconazole, miconozole, griseofulvin, hydroxyzine, diphenhydramine, pramoxine, lidocaine, procaine, mepivacaine, monobenzone, erythromycin, tetracycline, clindamycin, meclocyline, minocycline, hydroquinone, naproxen, ibuprofen, theophylline, cromolyn, albuterol, topical steroids (e.g., hydrocortisone, hydrocortisone 21-acetate, hydrocortisone 17-valerate, and hydrocortisone 17-butyrate), betamethasone valerate, betamethasone diproprionate, benzoyl peroxide, crotamiton, propranolol, promethazine, and mixtures or derivatives thereof. In a preferred, but not necessary embodiment of the present invention, the topical composition comprises one or more skin care actives selected from the group consisting of sunscreen agents, self-tanning agents, anti-aging agents, anti-wrinkle agents, anti-acne agents, antimicrobials, anti-inflammatory agents, skin-lightening agents, proteins or peptides, vitamins and derivatives thereof, exfoliants, ingredients that stimulate DNA repair, ingredients that provide immune protection, ingredients that stimulate cell renewal, ingredients that stimulate skin barrier repair, moisturizers, and mixtures thereof.

In a particularly preferred embodiment of the present invention, the topical or cosmetic composition is a sunscreen composition comprising composite particles containing core particles formed of zinc oxide, titanium dioxide, or both. As mentioned hereinabove, zinc oxide or titanium dioxide particles are known to have photoprotective characteristics and can therefore be used as physical sunscreen agents, but their uses in topical or cosmetic compositions are limited due to their photo-activity, i.e., their tendency to cause generation of reactive oxygen species upon exposure to UV light, which may degrade or otherwise interfere with certain organic cosmetic ingredients or skin care actives that are susceptible to oxidative decomposition or degradation. The treatment or modification of zinc oxide and/or titanium dioxide particles as described in the present invention effectively eliminates or reduces reactive oxygen species generated in the vicinity of such particles upon UV exposure, but without adversely affecting the sunscreen properties of such particles.

Consequently, the composite particles of the present invention containing zinc oxide and/or titanium dioxide can be ready formulated with organic cosmetic ingredients or skin care additives that are known to be susceptible to oxidative decomposition or degradation to form stable sunscreen compositions with significantly improved overall stability and prolonged shelf live. For example, the composite particles containing zinc oxide and/or titanium dioxide can be formulated with one or more organic dyes susceptible to oxidative decomposition or degradation to form color cosmetic compositions that also have sunscreen properties. For another example, the composite particles containing zinc oxide and/or titanium dioxide can be formulated with one or more organic sunscreen agents susceptible to oxidative decomposition or degradation, thereby forming sunscreen compositions that are not only characterized by high SPF values (e.g., SPF 30 or more), but also surprisingly and unexpectedly improved overall stability and prolonged shelf life. If present, such organic sunscreen agents may range from about 0.1 to 45% by weight of the total composition.

Exemplary organic sunscreen agents that can be used in combination with the TiO₂-and/or ZnO-containing composite particles of the present invention include, but are not limited to UVA and UVB sunscreens, such as benzophenones and derivatives thereof (e.g., benzophenone-3, dioxybenzone, sulisobenzone, octabenzone, hydroxy- and/or methoxy-substituted benzophenones, and benzophenonesulfonic acids and salts thereof); salicylic acid derivatives (e.g., ethylene glycol salicylate, triethanolamine salicylate, octyl salicylate, homomenthyl salicylate, and phenyl salicylate); urocanic acid and derivatives thereof (e.g., ethyl urocanate); p-aminobenzoic acid (PABA) and derivatives thereof (e.g., ethyl/isobutyl/glyceryl esters thereof and 2-ethylhexyl p-dimethylaminobenzoate, which is also referred to as octyldimethyl PABA); anthranilates and derivatives thereof (e.g., o-aminobenzoates and various esters of amino-benzoic acid); benzalmalonate derivatives; benzimidazole derivatives; imidazolines; bis-benzazolyl derivatives; dibenzoylmethanes and derivatives thereof (e.g., 4-tert-butyl-4'-methoxydibenzoylmethane, which is commonly referred to as "avobenzone," and 4-isopropyl-dibenzoylmethane); benzoazole/ benzodiazole/benzotriazoles and derivatives thereof (e.g., 2-(2-hydroxy-5-methylphenyl) benzotriazole and methylene bis-benzotriazolyl tetramethylbutylphenol, which is commonly referred to as "Tinosorb M"); diphenylacrylates and derivatives thereof (e.g., 2-ethylhexyl-2-cyano-3,3-diphenylacrylate, which is commonly referred to as "octocrylene," and ethyl-2-cyano-3,3-diphenylacrylate, which is commonly referred to as "etocrylene"); diesters or polyesters containing diphenylmethylene or 9H-fluorene substitutional groups; 2-phenyl-benzimidazole-5-sulphonic acid (PBSA); 4,4-diarylbutadienes; cinnamates and derivatives thereof (e.g., 2-ethylhexyl-p-methoxycinnamate, octyl-p-methoxycinnamate, umbelliferone, methylumbelliferone, methylaceto-umbelliferone, esculetin, methylesculetin, and daphnetin); camphors and derivatives thereof (e.g., 3-benzylidenecamphor, 4-methylbenzylidenecamphor, polyacrylamidomethyl benzylidenecamphor, benzylidene camphor sulfonic acid, and terephthalylidene dicamphor sulfonic acid, which is commonly referred to as "Encamsule"); triazines and derivatives thereof (e.g., 2,4-bis-{[4-(2-ethyl-hexyloxy)-2-hydroxy]-phenyl}-6-(4-methoxyphenyl)-1,3,5-triazine, which is commonly referred to as "Tinosorb S"); naphthalates and derivatives thereof (e.g., diethylhexyl-2,6-naphthalate); naphtholsulfonates and derivatives thereof (e.g., sodium salts of 2-naphthol-3,6-disulfonic and 2-naphthol-6,8-disulfonic acids); dibenzalacetone and benzalacetonephenone; diphenylbutadienes and derivatives thereof; di-hydroxynaphthoic acid and salts thereof; o- and p-hydroxybiphenyldisulfonates; coumarin derivatives (e.g., 7-hydroxy, 7-methyl, and 3-phenyl derivatives thereof); azoles/diazoles/triazoles and derivatives thereof (e.g., 2-acetyl-3-bromoindazole, phenyl benzoxazole, methyl naphthoxazole, and various aryl benzotriazoles); quinine and derivatives thereof (e.g., bisulfate, sulfate, chloride, oleate, and tannate salts thereof); quinoline and derivatives thereof (e.g., 2-phenylquinoline and 8-hydroxyquinoline salts); tannic acid and derivatives thereof (e.g., hexaethylether derivatives thereof); hydroquinone and derivatives thereof; uric acid and derivatives thereof; vilouric acid and derivatives thereof, and mixtures or combinations thereof. Salts and otherwise neutralized forms of certain acidic sunscreens from the list hereinabove are also useful herein. These organic sunscreen agents may be used alone or in combination of two or more. In addition, other known animal or vegetable extracts having UV light-absorbing ability may properly be used alone or in combination.

Organic sunscreen agents that are particularly useful for the practice of the present invention are: 4,4'-t-butyl methoxydibenzoylmethane, 2-ethylhexyl-2-cyano-3,3-diphenylacrylate, 2-ethylhexylsalicylate, 3,3,5-trimethylcyclohexylsalicylate, 2-ethylhexyl p-methoxycinnamate, 2-hydroxy-4-methoxybenzophenone, 2,2-dihydroxy-4-methoxybenzophenone, 2,4-bis-{4-(2-ethyl-hexyloxy)-2-hydroxy]-phenyl}-6-(4-methoxyphenyl)-1,3,5-triazine, methylene bis-benzotriazolyl tetramethylbutylphenol, terephthalylidene dicamphor sulfonic acid, diethylhexyl 2,6-naphthalate, digalloyltrioleate, ethyl 4-[bis(hydroxypropyl)]aminobenzoate, glycerol p-aminobenzoate, methylanthranilate, p-dimethylaminobenzoic acid or aminobenzoate, 2-ethylhexyl p-dimethylaminobenzoate, 2-phenylbenzimidazole-5-sulfonic acid, 2-(p-dimethylaminophenyl)-5-sulfoniobenzoxazoic acid, and mixtures or combinations thereof. Preferably, 4,4'-t-butyl methoxydibenzoylmethane is provided in the sunscreen compositions of the present invention, either with TiO₂-containing composite particles, or ZnO-containing composite particles, or both. More preferably, the sunscreen compositions of the present invention further include a second organic sunscreen agent selected from the lists provided hereinabove.

The cosmetically acceptable carrier may also contain one or more oils, which may be silicone, organic, or mixtures thereof. If present, such oils may range from about 0.1 to 99% by weight of the total composition and include volatile or non-volatile silicones such as cyclomethicone; methyl trimethicone; octamethyltrisiloxane; decamethyltetrasiloxane; dodecamethylpentasiloxane; dimethicone; phenyl trimethicone trimethylsiloxyphenyl dimethicone; phenyl dimethicone; cetyl dimethicone; dimethicone copolyol, cetyl dimethicone copolyol; glycerolated silicones such as lauryl PEG-9 polydimethylsiloxyethyl dimethicone; or mixtures thereof. Suitable esters include mono-, di-, or triesters of C4-30 fatty acids and mono-, di-, or polyhydric C1-20 alcohols, such as fatty acid (e.g., stearyl, behenyl, and isostearyl) esters of glycerin, or fatty acid esters of alpha hydroxyl acids such as citric, malic, or lactic acids and the like. Suitable hydrocarbons include monomeric or polymeric olefins or alpha olefins, such as polyisobutene, polydecene, polybutene, or hydrogenated derivatives thereof.

The cosmetically acceptable carrier may also comprise one or more humectants. If present, they may range from about 0.1 to 20% by weight of the total composition and include C1-4 alkylene glycols such as butylene, propylene, ethylene glycol, glycerin and the like.

The cosmetically acceptable carrier may also contain one or more waxes preferably having a melting point ranging from about 30 to 150° C. If present, such waxes may range from about 0.1 to 45% by weight of the total composition and include animal, vegetable, mineral, or silicone waxes. Examples include alkyl dimethicones stearyl dimethicone, candelilla, polyethylene, ozokerite, beeswax, and the like.

The cosmetically acceptable carrier may also comprise one or more organosiloxane elastomers, either emulsifying or non-emulsifying. If present, such elastomers may range from about 0.1 to 30% by weight of the total composition. Examples of suitable elastomers include dimethicone/vinyl dimethicone crosspolymer; dimethicone/dimethicone PEG/PPG 10/15 crosspolymer; and the like.

The cosmetically acceptable carrier may also include one or more pigments or powders or mixtures thereof. If present, the suggested ranges of such pigments or powders are from about 0.1 to 85% by weight of the total composition. The particle sizes of such pigments or powders may range from about 0.05 to 200 microns but are preferably about 50-100 microns. Examples of pigments include organic pigments such as D&C or FD&C colors or Lakes thereof including blues, browns, reds, etc; or inorganic iron oxides such as brown, yellow, green, red, iron oxides. Suitable powders include titanium dioxide, nylon, PMMA, boron nitride, mica, and the like.

The cosmetically acceptable carrier may also comprise one or more nonionic surfactants, particularly if the topical or cosmetic composition of the present invention is provided in the emulsion form. If present, such surfactants may range from about 0.1 to 20% by weight of the total composition. Suitable surfactants include ethoxylated fatty C6-30 alcohols such as steareth, beheneth, ceteth where the number following each of the surfactants refers to the number of repeating ethylene oxide groups which may range from 2 to 250, e.g. steareth-2, beheth-30 and so on.

## Claims

1. A composite particle comprising one or more core particles encapsulated within a polymeric shell, wherein at least some of the core particles comprise a material capable of releasing free oxygen radicals, wherein a first antioxidant capable of quenching or scavenging reactive oxygen species is co-encapsulated or co-entrapped with the core particles inside the polymeric shell, and wherein a second antioxidant capable of preventing or reducing oxidative damage to the skin is coated over the polymeric shell, and attached to said polymeric shell by either chemical or physical bonds, and wherein said polymeric shell is formed from a material selected from the group consisting of polyester, polyamide, polyphthalamide, polyimide, polycarbonate, polyketone, cellulose acetate, polysulfone, polyphenylene sulfide, polyphenylene oxides, polylactic acid, polyvinylpyrrolidone, polystyrene, polyacrylonitrile, polyacrylamide, polymethylmethacrylate, and polyacrylate.

2. The composite particle of claim 1, wherein at least some of the core particles comprise a metal oxide, said metal oxide preferably being selected from TiO₂ and ZnO.

3. The composite particle according to claims 1 or 2, wherein the first antioxidant is selected from the group consisting of singlet-oxygen scavengers, superoxide scavengers, hydroxyl radical scavengers, and mixtures or combinations thereof

4. The composite particle according to claims 1 or 2, wherein the first antioxidant is selected from the group consisting of *Acacia catechu* bark extract, alpha-tocopherol, anthocyanins, *Argania spinosa* leaf extract, aronia (chokeberry) extract, ascorbic acid and esters thereof, *Aspalathus linearis* (rooibos) extract, baicalin, bakuchiol, bamboo extract, bamboo leaf extract, benfotiamine, butylated hydroxytoluene (BHT), *Camellia sinensis* extract, catechins, chlorogenic acids, *Chondrus crispus* (carrageenan) extract, citrus peel extract, cocoa extract, cocoa polyphenols, cranberry extract, decarboxy carnosine HCl, diethylhexyl syringylidenel maloneate, epigallicathecin gallate (EGCG), ethylbisiminomethylguaiacol manganese chloride (EUK-134), ferulic acid, ferulic acid monophosphate, *Ficus carica* (common fig) extract, grape seed extract, grape skin extract, 4-hydroxy-isoleucine extract, 6-hydroxy-2,5,7,8-tetramethylchroman-2-carboxylic acid and phosphate thereof, hydroxy dimethoxybenzyl malonate, *Hypoxis hemerocallidea* (African potato) extract, idebenone, kaempferol, kiwi seed extract, kola nut (cola) powder, L-ergothioneine, L-histidine, lipoic acid and esters thereof, litchi seed extract, L-2-oxo-thiazolidine (OTZ), lutein, luteolin, manganese gluconate, mannitol, *Morinda citrifolia* (noni) leaf extract, myricetin, N-acetyl cysteine (NAC), pentagalloylglucose (PGG), *Phyllanthus emblica* (Indian gooseberry) extract, *Punica granatum* (pomegranate) extract, quercetin, quercitrin, red wine extract, resveratrol and derivatives thereof, *Ribes nigrum* (blackcurrent) extract, *Rhodiola rosea* (golden root) extract, *Rosa roxburghii* (chestnut rose) fruit extract, rosemary extract, rosemarinic acid, rutin, salicylic acid, salicyloyl cyclic phosphate, scutellaria extract, *Scutellaria baicaleszsis* root extract, *Siringa vulgaris* (Lilac) extract, spinach extract, tetrahydrocurcuminoids, tocopherol and esters thereof, vanilla extract, walnut polyphenols, preferably gamma-oryzanol, and mixtures or combinations thereof

5. The composite particle according to any one of claims 1-4, wherein the second antioxidant is selected from the group consisting of ascorbyl palmitate, ascorbyl stearate, butylated hydroxyanisole (BHA), butylated hydroxytoluene (BHT), *Caesalpinia paraensis* extract, chlorogenic acids, *Polypodium leucotomos* extract, *Acacia catechu* bark extract, alpha-tocopherol, anthocyanins, , *Argania spinosa* leaf extract, aronia (chokeberry) extract, ascorbic acid and esters thereof, ascorbyl tocopheryl maleate (2-CME), *Aspalathus linearis* (rooibos) extract, baicalin, bakuchiol, bamboo extract, bamboo leaf extract, benfotiamine, butylated hydroxytoluene (BHT), *Camellia sinensis* extract, *Chamomilla recutita* (matricaria) flower oil, catechins, chlorogenic acids, *Chondrus crispus* (carrageenan) extract, citrus peel extract, cocoa extract, cocoa polyphenols, cranberry extract, decarboxy carnosine HCl, diethylhexyl syringal malonate, epigallicathecin gallate (EGCG), ethylbisiminomethylguaiacol manganese chloride, ferulic acid, ferulic acid monophosphate, *Ficus carica* (common fig) extract, gamma-oryzanol, *Garcinia mangostana* peel extract, glucosylrutin, grape seed extract, grape skin extract, 4-hydroxy-isoleucine extract, 6-hydroxy-2,5,7,8-tetramethylchroman-2-carboxylic acid and phosphate thereof, hydroxy dimethoxybenzyl malonate, *Hypoxis hemerocallidea* (African potato) extract, idebenone, kaempferol, kiwi seed extract, kola nut (cola) powder, L-ergothioneine, L-histidine, Helianthus annuus (sunflower) seed extract, licorice extract, lipoic acid and esters thereof, litchi seed extract, L-2-oxo-thiazolidine (OTZ), lutein, luteolin, manganese gluconate, mannitol, *Mimosa tenuiflora* bark extract, *Morinda citrifolia* (noni) leaf extract, nordihydroguaiaretic acid (NDGA), myricetin, N-acetyl cysteine (NAC), *Nymphea alba* (water lily) flower extract, Oenothera biennis (evening primrose) seed extract, *Oryza sativa* (rice) extract, pentagalloylglucose (PGG), *Perilla ocymoides* seed extract, (*Phyllanthus emblica* (Indian gooseberry) extract, *Pimpinella anisum* (anise) fruit extract, *Pinus pinaster* (French maritime pine) bark extract, *Psoralea corylifolia* seed extract, *Punica granatum* (pomegranate) extract, quercetin, quercetin hydrate, quercitrin, red wine extract, resveratrol and derivatives thereof, *Ribes nigrum* (blackcurrent) extract, *Rhodiola rosea* (golden root) extract, *Rosa roxburghii* (chestnut rose) fruit extract, rosemary extract, rosemarinic acid, rutin, rutin hydrate, salicylic acid, salicyloyl cyclic phosphate, sodium azide, scutellaria extract, *Scutellaria baicaleszsis* root extract, *Siringa vulgaris* (Lilac) extract, spinach extract, tetrahydrocurcuminoids, *Thermus thermophillus* ferment, tocopherol and esters thereof, tocotrienols, ubiquinone, *Vaccinium myrtillis* (bilberry) extract, vanilla extract, walnut polyphenols, wasabi extract, xanthophyll, preferably ascorbyl tocopheryl maleate, and mixtures or combinations thereof

6. The composite particle according to any one of claims 1-5, further comprising at least one organic compound susceptible to oxidative decomposition or degradation, said organic compound preferably being selected from an organic dye or an organic sunscreen agent.

7. A topical composition comprising a dispersion of composite particles in a cosmetically or pharmaceutically acceptable medium, wherein each of said composite particles comprises one or more core particles encapsulated within a polymeric shell, wherein at least some of the core particles comprise a material capable of releasing reactive oxygen species, wherein a first antioxidant capable of quenching or scavenging reactive oxygen species is co-encapsulated or co-entrapped with the core particles inside the polymeric shell, and wherein a second antioxidant capable of preventing or reducing oxidative damage to the skin is coated over the polymeric shell, and attached to said polymeric shell by either chemical or physical bonds, and wherein said polymeric shell is formed from a material selected from the group consisting of polyester, polyamide, polyphthalamide, polyimide, polycarbonate, polyketone, cellulose acetate, polysulfone, polyphenylene sulfide, polyphenylene oxides, polylactic acid, polyvinylpyrrolidone, polystyrene, polyacrylonitrile, polyacrylamide, polymethylmethacrylate, and polyacrylate.

8. The composite particle of claim 7, wherein at least some of the core particles comprise a metal oxide, said metal oxide preferably being selected from TiO₂ and ZnO.

9. The topical composition of claim 7, wherein the first antioxidant is selected from the group consisting of singlet-oxygen scavengers, superoxide scavengers, hydroxyl radical scavengers, and mixtures or combinations thereof.

10. The topical composition according to claim 7, wherein the first antioxidant is selected from the group consisting of *Acacia catechu* bark extract, alpha-tocopherol, anthocyanins, *Argania spinosa* leaf extract, aronia (chokeberry) extract, ascorbic acid and esters thereof, *Aspalathus linearis* (rooibos) extract, baicalin, bakuchiol, bamboo extract, bamboo leaf extract, benfotiamine, butylated hydroxytoluene (BHT), *Camellia sinensis* extract, catechins, chlorogenic acids, *Chondrus crispus* (carrageenan) extract, citrus peel extract, cocoa extract, cocoa polyphenols, cranberry extract, decarboxy carnosine HCl, diethylhexyl syringylidenel maloneate, epigallicathecin gallate (EGCG), ethylbisiminomethylguaiacol manganese chloride (EUK-134), ferulic acid, ferulic acid monophosphate, *Ficus carica* (common fig) extract, grape seed extract, grape skin extract, 4-hydroxy-isoleucine extract, 6-hydroxy-2,5,7,8-tetramethylchroman-2-carboxylic acid and phosphate thereof, hydroxy dimethoxybenzyl malonate, *Hypoxis hemerocallidea* (African potato) extract, idebenone, kaempferol, kiwi seed extract, kola nut (cola) powder, L-ergothioneine, L-histidine, lipoic acid and esters thereof, litchi seed extract, L-2-oxo-thiazolidine (OTZ), lutein, luteolin, manganese gluconate, mannitol, *Morinda citrifolia* (noni) leaf extract, myricetin, N-acetyl cysteine (NAC), pentagalloylglucose (PGG), *Phyllanthus emblica* (Indian gooseberry) extract, *Punica granatum* (pomegranate) extract, quercetin, quercitrin, red wine extract, resveratrol and derivatives thereof, *Ribes nigrum* (blackcurrent) extract, *Rhodiola rosea* (golden root) extract, *Rosa roxburghii* (chestnut rose) fruit extract, rosemary extract, rosemarinic acid, rutin, salicylic acid, salicyloyl cyclic phosphate, scutellaria extract, *Scutellaria baicaleszsis* root extract, *Siringa vulgaris* (Lilac) extract, spinach extract, tetrahydrocurcuminoids, tocopherol and esters thereof, vanilla extract, walnut polyphenols, preferably gamma-oryzanol, and mixtures or combinations thereof

11. The topical composition according to any one of claims 7 to 10, wherein the second antioxidant is selected from the group consisting of ascorbyl palmitate, ascorbyl stearate, butylated hydroxyanisole (BHA), butylated hydroxytoluene (BHT), *Caesalpinia paraensis* extract, chlorogenic acids, *Polypodium leucotomos* extract, *Acacia catechu* bark extract, alpha-tocopherol, anthocyanins, *Argania spinosa* leaf extract, aronia (chokeberry) extract, ascorbic acid and esters thereof, ascorbyl tocopheryl maleate (2-CME), *Aspalathus linearis* (rooibos) extract, baicalin, bakuchiol, bamboo extract, bamboo leaf extract, benfotiamine, butylated hydroxytoluene (BHT), *Camellia sinensis* extract, *Chamomilla recutita* (matricaria) flower oil, catechins, chlorogenic acids, *Chondrus crispus* (carrageenan) extract, citrus peel extract, cocoa extract, cocoa polyphenols, cranberry extract, decarboxy carnosine HCl, diethylhexyl syringal malonate, epigallicathecin gallate (EGCG), ethylbisiminomethylguaiacol manganese chloride, ferulic acid, ferulic acid monophosphate, *Ficus carica* (common fig) extract, gamma-oryzanol, *Garcinia mangostana* peel extract, glucosylrutin, grape seed extract, grape skin extract, 4-hydroxy-isoleucine extract, 6-hydroxy-2,5,7,8-tetramethylchroman-2-carboxylic acid and phosphate thereof, hydroxy dimethoxybenzyl malonate, *Hypoxis hemerocallidea* (African potato) extract, idebenone, kaempferol, kiwi seed extract, kola nut (cola) powder, L-ergothioneine, L-histidine, Helianthus annuus (sunflower) seed extract, licorice extract, lipoic acid and esters thereof, litchi seed extract, L-2-oxo-thiazolidine (OTZ), lutein, luteolin, manganese gluconate, mannitol, *Mimosa tenuiflora* bark extract, *Morinda citrifolia* (noni) leaf extract, nordihydroguaiaretic acid (NDGA), myricetin, N-acetyl cysteine (NAC), *Nymphea alba* (water lily) flower extract, Oenothera biennis (evening primrose) seed extract, *Oryza sativa* (rice) extract, pentagalloylglucose (PGG), *Perilla ocymoides* seed extract, (*Phyllanthus emblica* (Indian gooseberry) extract, *Pimpinella anisum* (anise) fruit extract, *Pinus pinaster* (French maritime pine) bark extract, *Psoralea corylifolia* seed extract, *Punica granatum* (pomegranate) extract, quercetin, quercetin hydrate, quercitrin, red wine extract, resveratrol and derivatives thereof, *Ribes nigrum* (blackcurrent) extract, *Rhodiola rosea* (golden root) extract, *Rosa roxburghii* (chestnut rose) fruit extract, rosemary extract, rosemarinic acid, rutin, rutin hydrate, salicylic acid, salicyloyl cyclic phosphate, sodium azide, scutellaria extract, *Scutellaria baicaleszsis* root extract, *Siringa vulgaris* (Lilac) extract, spinach extract, tetrahydrocurcuminoids, *Thermus thermophillus* ferment, tocopherol and esters thereof, tocotrienols, ubiquinone, *Vaccinium myrtillis* (bilberry) extract, vanilla extract, walnut polyphenols, wasabi extract, xanthophyll, preferably ascorbyl tocopheryl maleate, and mixtures or combinations thereof

12. The topical composition according to any one of claims 7 to 11, further comprising at least one organic compound susceptible to oxidative decomposition or degradation, said organic compound preferably being selected from an organic dye and an organic sunscreen agent.

## Patentansprüche

1. Verbundstoffteilchen, umfassend ein oder mehrere Kernteilchen, die in einer polymeren Hülle eingekapselt sind, wobei mindestens einige der Kernteilchen ein Material umfassen, das fähig ist, freie Sauerstoffradikale freizusetzen, wobei ein erstes Antioxidans, das fähig ist, reaktive Sauerstoffspezies zu vernichten oder abzufangen, mit den Kernteilchen im Inneren der polymeren Hülle gemeinsam eingekapselt oder gemeinsam eingeschlossen ist und wobei ein zweites Antioxidans, das fähig ist, oxidative Schädigung der Haut zu verhindern oder zu mindern, über der polymeren Hülle aufgetragen ist und entweder durch chemische oder physikalische Bindungen an der polymeren Hülle befestigt ist und wobei die polymere Hülle aus einem Material gebildet ist, das aus der Gruppe ausgewählt ist, die aus Polyester, Polyamid, Polyphthalamid, Polyimid, Polycarbonat, Polyketon, Celluloseacetat, Polysulfon, Polyphenylensulfid, Polyphenylenoxiden, Polymilchsäure, Polyvinylpyrrolidon, Polystyrol, Polyacrylnitril, Polyacrylamid, Polymethylmethacrylat und Polyacrylat besteht.

2. Verbundstoffteilchen nach Anspruch 1, wobei mindestens einige der Kernteilchen ein Metalloxid umfassen, wobei das Metalloxid vorzugsweise aus TiO₂ und ZnO ausgewählt ist.

3. Verbundstoffteilchen nach Anspruch 1 oder 2, wobei das erste Antioxidans aus der Gruppe ausgewählt ist, die aus Singulett-Sauerstoff-Fängern, Superoxidfängern, Hydroxylradikalfängern und Gemischen oder Kombinationen davon besteht.

4. Verbundstoffteilchen nach Anspruch 1 oder 2, wobei das erste Antioxidans aus der Gruppe ausgewählt ist, die aus Rindenextrakt von *Acacia catechu*, alpha-Tocopherol, Anthocyaninen, Blattextrakt von *Argania spinosa,* Extrakt von Aronia (Apfelbeere), Ascorbinsäure und Estern davon, Extrakt von *Aspalathus linearis* (Rotbusch), Baicalin, Bakuchiol, Bambusextrakt, Bambusblattextrakt, Benfotiamin, butyliertem Hydroxytoluol (BHT), Extrakt von *Camellia sinensis,* Catechinen, Chlorogensäuren, Extrakt von *Chondrus crispus* (Carrageenan), Zitrusschalenextrakt, Kakaoextrakt, Kakaopolyphenolen, Preiselbeerextrakt, Decarboxycarnosin-HCl, Diethylhexylsyringylidenmalonat, Epigallocatechin-gallat (EGCG), Ethylbisiminomethylguajacol-manganchlorid (EUK-134), Ferulasäure, Ferulasäuremonophosphat, Extrakt von *Ficus carica* (Gemeine Feige), Traubenkernextrakt, Traubenschalenextrakt, 4-Hydroxyisoleucin-Extrakt, 6-Hydroxy-2,5,7,8-tetramethylchroman-2-carbonsäure und Phosphat davon, Hydroxydimethoxybenzylmalonat, Extrakt von *Hypoxis hemerocallidea* (afrikanische Kartoffel), Idebenon, Kampferöl, Kiwisamenextrakt, Pulver der Kolanuss (Cola), L-Ergothionein, L-Histidin, Liponsäure und Estern davon, Litschisamenextrakt, L-2-Oxo-thiazolidin (OTZ), Lutein, Luteolin, Mangangluconat, Mannit, Blattextrakt von *Morinda citrifolia* (Noni), Myricetin, N-Acetylcystein (NAC), Pentagalloylglucose (PGG), Extrakt von *Phyllanthus emblica* (Indische Stachelbeere), Extrakt von *Punica granatum* (Granatapfel), Quercetin, Quercitrin, Rotweinextrakt, Resveratrol und Derivaten davon, Extrakt von *Ribes nigrum* (Schwarze Johannisbeere), Extrakt von *Rhodiola rosea* (Rosenwurz), Fruchtextrakt von *Rosa roxburghii* (Kastanienrose), Rosmarinextrakt, Rosmarinsäure, Rutin, Salicylsäure, Salicyloylcyclophosphat, Scutellaria-Extrakt, Wurzelextrakt von *Scutellaria baicaleszsis,* Extrakt von *Siringa vulgaris* (Flieder), Spinatextrakt, Tetrahydro-curcuminoiden, Tocopherol und Estern davon, Vanilleextrakt, Walnusspolyphenolen, vorzugsweise gamma-Oryzanol, und Gemischen und Kombinationen davon besteht.

5. Verbundstoffteilchen nach einem von Anspruch 1 bis 4, wobei das zweite Antioxidans aus der Gruppe ausgewählt ist, die aus Ascorbylpalmitat, Ascorbylstearat, butyliertem Hydroxyanisol (BHA), butyliertem Hydroxytoluol (BHT), Extrakt von *Caesalpinia paraensis,* Chlorogensäuren, Extrakt von *Polypodium leucotomos*, Rindenextrakt von *Acacia catechu*, alpha-Tocopherol, Anthocyaninen, Blattextrakt von *Argania spinosa,* Extrakt von Aronia (Apfelbeere), Ascorbinsäure und Estern davon, Ascorbyltocopherylmaleat (2-CME), Extrakt von *Aspalathus linearis* (Rotbusch), Baicalin, Bakuchiol, Bambusextrakt, Bambusblattextrakt, Benfotiamin, butyliertem Hydroxytoluol (BHT), Extrakt von *Camellia sinensis,* Blütenöl von *Chamomilla recutita* (Matricaria), Catechinen, Chlorogensäuren, Extrakt von *Chondrus crispus* (Carrageenan), Zitrusschalenextrakt, Kakaoextrakt, Kakaopolyphenolen, Preiselbeerextrakt, Decarboxycarnosin-HCl, Diethylhexylsyringalmalonat, Epigallocatechingallat (EGCG), Ethylbisiminomethylguajacolmangan-chlorid, Ferulasäure, Ferulasäuremonophosphat, Extrakt von *Ficus carica* (Gemeine Feige), gamma-Oryzanol, Schalen-extrakt von *Garcinia mangostana,* Glucosylrutin, Traubenkernextrakt, Traubenschalenextrakt, 4-Hydroxy-isoleucin-Extrakt, 6-Hydroxy-2,5,7,8-tetramethylchroman-2-carbonsäure und Phosphat davon, Hydroxydimethoxy-benzylmalonat, Extrakt von *Hypoxis hemerocallidea* (afrikanische Kartoffel), Idebenon, Kaempferöl, Kiwisamenextrakt, Pulver der Kolanuss (Cola), L-Ergothionein, L-Histidin, Samenextrakt von Helianthus annuus (Sonnenblume), Süßholzextrakt, Liponsäure und Estern davon, Litschisamenextrakt, L-2-Oxo-thiazolidin (OTZ), Lutein, Luteolin, Mangangluconat, Mannit, Rindenextrakt von *Mimosa tenuiflora,* Blattextrakt von *Morinda citrifolia* (Noni), Nordihydroguajaretsäure (NDGA), Myricetin, N-Acetylcystein (NAC), Blütenextrakt von *Nymphea alba* (Wasserlilie), Samenextrakt von Oenothera biennis (Gemeine Nachtkerze), Extrakt von *Oryza sativa* (Reis), Pentagalloylglucose (PGG), Samenextrakt von *Perilla ocymoides,* Extrakt von *Phyllanthus emblica* (Indische Stachelbeere), Fruchtextrakt von *Pimpinella anisum* (Anis), Rindenextrakt von *Pinus pinaster* (Strandkiefer), Samenextrakt von *Psoralea corylifolia,* Extrakt von *Punica granatum* (Granatapfel), Quercetin, Quercetinhydrat, Quercitrin, Rotweinextrakt, Resveratrol und Derivaten davon, Extrakt von *Ribes nigrum* (Schwarze Johannisbeere), Extrakt von *Rhodiola rosea* (Rosenwurz), Fruchtextrakt von *Rosa roxburghii* (Kastanienrose), Rosmarinextrakt, Rosmarin-säure, Rutin, Rutinhydrat, Salicylsäure, Salicyloyl-cyclophosphat, Natriumazid, Scutellaria-Extrakt, Wurzelextrakt von Scutellaria baicaleszsis, Extrakt von *Siringa vulgaris* (Flieder), Spinatextrakt, Tetrahydrocurcuminoiden, *Thermusthermo-philus-*Ferment, Tocopherol und Estern davon, Tocotrienolen, Ubichinon, Extrakt von *Vaccinium myrtillis* (Heidelbeere), Vanilleextrakt, Walnusspolyphenolen, Wasabiextrakt, Xanthophyll, vorzugsweise Ascorbyltoco-pherylmaleat, und Gemischen oder Kombinationen davon besteht.

6. Verbundstoffteilchen nach einem von Anspruch 1 bis 5, ferner umfassend mindestens eine organische Verbindung, die gegen oxidative Zersetzung oder oxidativen Abbau empfindlich ist, wobei die organische Verbindung vorzugsweise aus einem organischen Farbstoff oder einem organischen Sonnenschutzmittel ausgewählt ist.

7. Topische Zusammensetzung, umfassend eine Dispersion von Verbundstoffteilchen in einem kosmetisch oder pharmazeutisch akzeptablen Medium, wobei jedes der Verbundstoffteilchen ein oder mehrere Kernteilchen umfasst, die in einer polymeren Hülle eingekapselt sind, wobei mindestens einige der Kernteilchen ein Material umfassen, das fähig ist, reaktive Sauerstoffspezies freizusetzen, wobei ein erstes Antioxidans, das fähig ist, reaktive Sauerstoffspezies zu vernichten oder abzufangen, mit den Kernteilchen im Inneren der polymeren Hülle gemeinsam eingekapselt oder gemeinsam eingeschlossen ist und wobei ein zweites Antioxidans, das fähig ist, oxidative Schädigung der Haut zu verhindern oder zu mindern, über der polymeren Hülle aufgetragen ist und entweder durch chemische oder physikalische Bindungen an der polymeren Hülle befestigt ist und wobei die polymere Hülle aus einem Material gebildet ist, das aus der Gruppe ausgewählt ist, die aus Polyester, Polyamid, Polyphthalamid, Polyimid, Polycarbonat, Polyketon, Celluloseacetat, Polysulfon, Polyphenylensulfid, Polyphenylenoxiden, Polymilchsäure, Polyvinylpyrrolidon, Polystyrol, Polyacrylnitril, Poly-acrylamid, Polymethylmethacrylat und Polyacrylat besteht.

8. Verbundstoffteilchen nach Anspruch 7, wobei mindestens einige der Kernteilchen ein Metalloxid umfassen, wobei das Metalloxid vorzugsweise aus TiO₂ und ZnO ausgewählt ist.

9. Topische Zusammensetzung nach Anspruch 7, wobei das erste Antioxidans aus der Gruppe ausgewählt ist, die aus Singulett-Sauerstoff-Fängern, Superoxidfängern, Hydroxyl-radikalfängern und Gemischen oder Kombinationen davon besteht.

10. Topische Zusammensetzung nach Anspruch 7, wobei das erste Antioxidans aus der Gruppe ausgewählt ist, die aus Rindenextrakt von *Acacia catechu*, alpha-Tocopherol, Anthocyaninen, Blattextrakt von *Argania spinosa,* Extrakt von Aronia (Apfelbeere), Ascorbinsäure und Estern davon, Extrakt von *Aspalathus linearis* (Rotbusch), Baicalin, Bakuchiol, Bambusextrakt, Bambusblattextrakt, Benfotiamin, butyliertem Hydroxytoluol (BHT), Extrakt von *Camellia sinensis,* Catechinen, Chlorogensäuren, Extrakt von *Chondrus crispus* (Carrageenan), Zitrusschalenextrakt, Kakaoextrakt, Kakaopolyphenolen, Preiselbeerextrakt, Decarboxycarnosin-HCl, Diethylhexylsyringylidenmaloneat, Epigallocatechin-gallat (EGCG), Ethylbisiminomethylguajacol-manganchlorid (EUK-134), Ferulasäure, Ferulasäuremonophosphat, Extrakt von *Ficus carica* (Gemeine Feige), Traubenkernextrakt, Traubenschalenextrakt, 4-Hydroxyisoleucin-Extrakt, 6-Hydroxy-2,5,7,8-tetramethylchroman-2-carbonsäure und Phosphat davon, Hydroxydimethoxybenzylmalonat, Extrakt von *Hypoxis hemerocallidea* (afrikanische Kartoffel), Idebenon, Kaempferol, Kiwisamenextrakt, Pulver der Kolanuss (Cola), L-Ergothionein, L-Histidin, Liponsäure und Estern davon, Litschisamenextrakt, L-2-Oxo-thiazolidin (OTZ), Lutein, Luteolin, Mangangluconat, Mannit, Blattextrakt von *Morinda citrifolia* (Noni), Myricetin, N-Acetylcystein (NAC), Pentagalloylglucose (PGG), Extrakt von *Phyllanthus emblica* (Indische Stachelbeere), Extrakt von *Punica granatum* (Granatapfel), Quercetin, Quercitrin, Rotweinextrakt, Resveratrol und Derivaten davon, Extrakt von *Ribes nigrum* (Schwarze Johannisbeere), Extrakt von *Rhodiola rosea* (Rosenwurz), Fruchtextrakt von *Rosa roxburghii* (Kastanienrose), Rosmarinextrakt, Rosmarinsäure, Rutin, Salicylsäure, Salicyloylcyclophosphat, Scutellaria-Extrakt, Wurzelextrakt von *Scutellaria baicaleszsis,* Extrakt von *Siringa vulgaris* (Flieder), Spinatextrakt, Tetrahydro-curcuminoiden, Tocopherol und Estern davon, Vanilleextrakt, Walnusspolyphenolen, vorzugsweise gamma-Oryzanol, und Gemischen und Kombinationen davon besteht.

11. Topische Zusammensetzung nach einem von Anspruch 7 bis 10, wobei das zweite Antioxidans aus der Gruppe ausgewählt ist, die aus Ascorbylpalmitat, Ascorbylstearat, butyliertem Hydroxyanisol (BHA), butyliertem Hydroxytoluol (BHT), Extrakt von *Caesalpinia paraensis,* Chlorogensäuren, Extrakt von *Polypodium leucotomos*, Rindenextrakt von *Acacia catechu*, alpha-Tocopherol, Anthocyaninen, Blattextrakt von *Argania spinosa,* Extrakt von Aronia (Apfelbeere), Ascorbinsäure und Estern davon, Ascorbyltocopherylmaleat (2-CME), Extrakt von *Aspalathus linearis* (Rotbusch), Baicalin, Bakuchiol, Bambusextrakt, Bambusblattextrakt, Benfotiamin, butyliertem Hydroxytoluol (BHT), Extrakt von *Camellia sinensis,* Blütenöl von *Chamomilla recutita* (Matricaria), Catechinen, Chlorogensäuren, Extrakt von *Chondrus crispus* (Carrageenan), Zitrusschalenextrakt, Kakaoextrakt, Kakaopolyphenolen, Preiselbeerextrakt, Decarboxycarnosin-HCl, Diethylhexylsyringalmalonat, Epigallocatechingallat (EGCG), Ethylbisiminomethylguajacol-manganchlorid, Ferulasäure, Ferulasäuremonophosphat, Extrakt von *Ficus carica* (Gemeine Feige), gamma-Oryzanol, Schalenextrakt von *Garcinia mangostana*, Glucosylrutin, Traubenkernextrakt, Traubenschalenextrakt, 4-Hydroxyiso-leucin-Extrakt, 6-Hydroxy-2,5,7,8-tetramethylchroman-2-carbonsäure und Phosphat davon, Hydroxydimethoxybenzylmalonat, Extrakt von *Hypoxis hemerocallidea* (afrikanische Kartoffel), Idebenon, Kaempferol, Kiwisamenextrakt, Pulver der Kolanuss (Cola), L-Ergothionein, L-Histidin, Samenextrakt von Helianthus annuus (Sonnenblume), Süßholzextrakt, Liponsäure und Estern davon, Litschisamenextrakt, L-2-Oxo-thiazolidin (OTZ), Lutein, Luteolin, Mangangluconat, Mannit, Rindenextrakt von *Mimosa tenuiflora,* Blattextrakt von *Morinda citrifolia* (Noni), Nordihydroguajaretsäure (NDGA), Myricetin, N-Acetylcystein (NAC), Blütenextrakt von *Nymphea alba* (Wasserlilie), Samenextrakt von Oenothera biennis (Gemeine Nachtkerze), Extrakt von *Oryza sativa* (Reis), Pentagalloylglucose (PGG), Samenextrakt von *Perilla ocymoides,* Extrakt von *Phyllanthus emblica* (Indische Stachelbeere), Fruchtextrakt von *Pimpinella anisum* (Anis), Rindenextrakt von *Pinus pinaster* (Strandkiefer), Samenextrakt von *Psoralea corylifolia,* Extrakt von *Punica granatum* (Granatapfel), Quercetin, Quercetinhydrat, Quercitrin, Rotweinextrakt, Resveratrol und Derivaten davon, Extrakt von *Ribes nigrum* (Schwarze Johannisbeere), Extrakt von *Rhodiola rosea* (Rosenwurz), Fruchtextrakt von *Rosa roxburghii* (Kastanienrose), Rosmarinextrakt, Rosmarinsäure, Rutin, Rutinhydrat, Salicylsäure, Salicyloylcyclophosphat, Natriumazid, Scutellaria-Extrakt, Wurzelextrakt von *Scutellaria baicaleszsis,* Extrakt von *Siringa vulgaris* (Flieder), Spinatextrakt, Tetrahydrocurcuminoiden, *Thermusthermophilus-*Ferment, Tocopherol und Estern davon, Tocotrienolen, Ubichinon, Extrakt von *Vaccinium myrtillis* (Heidelbeere), Vanille-extrakt, Walnusspolyphenolen, Wasabiextrakt, Xanthophyll, vorzugsweise Ascorbyltocopherylmaleat, und Gemischen oder Kombinationen davon besteht.

12. Topische Zusammensetzung nach einem von Anspruch 7 bis 11, ferner umfassend mindestens eine organische Verbindung, die gegen oxidative Zersetzung oder oxidativen Abbau empfindlich ist, wobei die organische Verbindung vorzugsweise aus einem organischen Farbstoff und einem organischen Sonnenschutzmittel ausgewählt ist.

## Revendications

1. Particule composite comprenant une ou plusieurs particules centrales encapsulées dans une coque polymère, dans laquelle au moins certaines des particules centrales comprennent un matériau capable de libérer des radicaux libres oxygène, dans laquelle un premier antioxydant capable de désactiver ou de piéger les espèces réactives de l'oxygène est co-encapsulé ou co-piégé avec les particules centrales à l'intérieur de la coque polymère, et dans laquelle un second antioxydant capable de prévenir ou de réduire les dommages oxydatifs occasionnés à la peau est appliqué sur la coque polymère, et lié à ladite coque polymère par des liaisons soit chimiques, soit physiques, et dans laquelle ladite coque polymère est formée à partir d'un matériau choisi dans le groupe constitué par un polyester, polyamide, polyphtalamide, polyimide, polycarbonate, polycétone, acétocellulose, polysulfone, polysulfure de phénylène, poly(oxydes de phénylène), acide polylactique, polyvinylpyrrolidone, polystyrène, polyacrylonitrile, polyacrylamide, polyméthacrylate de méthyle, et polyacrylate.

2. Particule composite selon la revendication 1, dans laquelle au moins certaines des particules centrales comprennent un oxyde métallique, ledit oxyde métallique étant de préférence choisi parmi TiO₂ et ZnO.

3. Particule composite selon les revendications 1 ou 2, dans laquelle le premier antioxydant est choisi dans le groupe constitué par les pièges à oxygène singulet, les pièges à superoxydes, les pièges à radicaux hydroxyle, et leurs mélanges ou combinaisons.

4. Particule composite selon les revendications 1 ou 2, dans laquelle le premier antioxydant est choisi dans le groupe constitué par un extrait d'écorce d'*Acacia catechu*, l'alpha-tocophérol, les anthocyanines, un extrait de feuilles d'*Argania spinosa,* un extrait d'*Aronia* (aronia), l'acide ascorbique et ses esters, un extrait d'*Aspalathus linearis* (rooibos), la baïcaline, le bakuchiol, un extrait de bambou, un extrait de feuilles de bambou, la benfotiamine, l'hydroxytoluène butylé (BHT), un extrait de *Came*/*lia sinensis,* les catéchines, les acides chlorogéniques, un extrait de *Chondrus crispus* (carraghénane), un extrait d'écorces d'agrumes, un extrait de cacao, les polyphénols de cacao, un extrait de canneberge, le décarboxycarnosine HCl, le syringylidène malonate de diéthylhexyle, le gallate d'épigallicathécine (EGCG), le chlorure d'éthylbisiminométhylguaiacol manganèse (EUK-134), l'acide férulique, le monophosphate d'acide férulique, un extrait de *Ficus carica* (figue commune), un extrait de pépins de raisin, un extrait de peau de raisin, un extrait de 4-hydroxy-isoleucine, l'acide 6-hydroxy-2,5,7,8-tétraméthylchromane-2-carboxylique et son phosphate, le malonate d'hydroxydiméthoxybenzyle, un extrait d'*Hypoxis hemerocallidea* (pomme de terre africaine), l'idébénone, le kaempférol, un extrait de graines de kiwi, la poudre de noix de kola (cola), la L-ergothionéine, la L-histidine, l'acide lipoïque et ses esters, un extrait de graines de litchi, la L-2-oxo-thiazolidine (OTZ), la lutéine, la lutéoline, le gluconate de manganèse, le mannitol, un extrait de feuilles de *Morinda citrifolia* (noni), la myricétine, la N-acétyl-cystéine (NAC), le pentagalloylglucose (PGG), un extrait de *Phyllantus emblica* (groseille à maquereau indienne), un extrait de *Punica granatum* (grenade), la quercétine, la quercitrine, un extrait de vin rouge, le resvératrol et ses dérivés, un extrait de *Ribes nigrum* (cassis), un extrait de *Rhodiola rosea* (racine d'or), un extrait de fruit de *Rosa roxburghii* (Chesnut rose), un extrait de romarin, l'acide rosmarinique, la rutine, l'acide salicylique, le phosphate cyclique de salicyloyle, un extrait de *Scutellaria,* un extrait de racines de *Scutellaria baicaleszsis,* un extrait de *Siringa vulgaris* (lilas), un extrait d'épinard, les tétrahydrocurcuminoïdes, le tocophérol et ses esters, un extrait de vanille, les polyphénols du noyer, de préférence le gamma-oryzanol, et leurs mélanges ou leurs combinaisons.

5. Particule composite selon l'une quelconque des revendications 1 à 4, dans laquelle le second antioxydant est choisi dans le groupe constitué par le palmitate d'ascorbyle, le stéarate d'ascorbyle, l'hydroxyanisole butylé (BHA), l'hydroxytoluène butylé (BHT), un extrait de *Caesalpinia paraensis,* les acides chlorogéniques, un extrait de *Polypodium leucotomos*, un extrait d'écorce d'*Acacia catechu,* l'alpha-tocophérol, les anthocyanines, un extrait de feuilles d'*Argania spinosa,* un extrait d'*Aronia* (aronia), l'acide ascorbique et ses esters, le maléate d'ascorbyltocophéryle (2-CME), un extrait d'*Aspalathus linearis* (rooibos), la baïcaline, le bakuchiol, un extrait de bambou, un extrait de feuilles de bambou, la benfotiamine, l'hydroxytoluène butylé (BHT), un extrait de *Came*/*lia sinensis,* l'huile de fleurs de *Chamomilla recutita* (matricaire), les catéchines, les acides chlorogéniques, un extrait de *Chondrus crispus* (carraghénane), un extrait d'écorces d'agrumes, un extrait de cacao, les polyphénols de cacao, un extrait de canneberge, la décarboxycarnosine HCl, le syringylidène malonate de diéthylhexyle, le gallate d'épigallicathécine (EGCG), le chlorure d'éthylbisiminométhylguaiacol manganèse, l'acide férulique, le monophosphate d'acide férulique, un extrait de *Ficus carica* (figue commune), le gamma-oryzanol, un extrait de peau de *Garcinia mangostana*, la glucosylrutine, un extrait de pépins de raisin, un extrait de peau de raisin, un extrait de 4-hydroxy-isoleucine, l'acide 6-hydroxy-2,5,7,8-tétraméthylchromane-2-carboxylique et son phosphate, le malonate d'hydroxydiméthoxybenzyle, un extrait d'*hypoxis hemerocallidea* (pomme de terre africaine), l'idébénone, le kaempférol, un extrait de graines de kiwi, la poudre de noix de kola (cola), la L-ergothionéine, la L-histidine, un extrait de graines d'*Helianthus annuus* (tournesol), un extrait de réglisse, l'acide lipoïque et ses esters, un extrait de graines de litchi, la L-2-oxo-thiazolidine (OTZ), la lutéine, la lutéoline, le gluconate de manganèse, le mannitol, un extrait d'écorce de *Mimosa tenuiflora,* un extrait de feuilles de *Morinda citrifolia* (noni), l'acide nordihydroguaiarétique (NDGA), la myricétine, la N-acétyl-cystéine (NAC), un extrait de fleur de *Nymphea alba* (nénuphar), un extrait de graines d'*Oenothera biennis* (onagre), un extrait d'*Oryza sativa* (riz), le pentagalloylglucose (PGG), un extrait de graines de *Perilla ocymoides,* un extrait de *Phyllantus emblica* (groseille à maquereau indienne), un extrait de fruit de *Pimpinella anisum* (anis vert), un extrait d'écorces de *Pinus pinaster* (pin maritime français), un extrait de graines de *Psoralea corylifolia,* un extrait de *Punica granatum* (grenade), la quercétine, l'hydrate de quercétine, la quercitrine, un extrait de vin rouge, le resvératrol et ses dérivés, un extrait de *Ribes nigrum* (cassis), un extrait de *Rhodiola rosea* (racine d'or), un extrait de fruit de *Rosa roxburghii* (Chesnut rose), un extrait de romarin, l'acide rosmarinique, la rutine, l'hydrate de rutine, l'acide salicylique, le phosphate cyclique de salicyloyle, l'azoture de sodium, un extrait de *Scutellaria,* un extrait de racines de *Scutellaria baicaleszsis,* un extrait de *Siringa vulgaris* (lilas), un extrait d'épinard, les tétrahydro-curcuminoïdes, un ferment de *Thermus thermophillus,* le tocophérol et ses esters, les tocotriénols, l'ubiquinone, un extrait de *Vaccinium myrtillis* (myrtille), un extrait de vanille, les polyphénols du noyer, un extrait de wasabi, une xanthophylle, de préférence le maléate d'ascorbyltocophéryle, et leurs mélanges ou leurs combinaisons.

6. Particule composite selon l'une quelconque des revendications 1 à 5, comprenant en outre au moins un composé organique sensible à la décomposition ou à la dégradation oxydative, ledit composé organique étant de préférence choisi parmi un colorant organique ou un agent écran solaire organique.

7. Composition à usage topique comprenant une dispersion de particules composites dans un milieu acceptable sur le plan cosmétique ou pharmaceutique, dans laquelle chacune desdites particules composites comprend une ou plusieurs particules centrales encapsulées dans une coque polymère, dans laquelle au moins certaines des particules centrales comprennent un matériau capable de libérer des radicaux libres oxygène, dans laquelle un premier antioxydant capable de désactiver ou de piéger les espèces réactives de l'oxygène est co-encapsulé ou co-piégé avec les particules centrales à l'intérieur de la coque polymère, et dans laquelle un second antioxydant capable de prévenir ou de réduire les dommages oxydatifs occasionnés à la peau est appliqué sur la coque polymère, et lié à ladite coque polymère par des liaisons soit chimiques, soit physiques, et dans laquelle ladite coque polymère est formée à partir d'un matériau choisi dans le groupe constitué par un polyester, polyamide, polyphtalamide, polyimide, polycarbonate, polycétone, acétocellulose, polysulfone, polysulfure de phénylène, poly(oxydes de phénylène), acide polylactique, polyvinylpyrrolidone, polystyrène, polyacrylonitrile, polyacrylamide, polyméthacrylate de méthyle, et polyacrylate.

8. Particule composite selon la revendication 7, dans laquelle au moins certaines des particules centrales comprennent un oxyde métallique, ledit oxyde métallique étant de préférence choisi parmi le TiO₂ et le ZnO.

9. Composition à usage topique selon la revendication 7, dans laquelle le premier antioxydant est choisi dans le groupe constitué par les pièges à oxygène singulet, les pièges à superoxydes, les pièges à radicaux hydroxyle, et leurs mélanges ou combinaisons.

10. Composition à usage topique selon la revendication 7, dans laquelle le premier antioxydant est choisi dans le groupe constitué par un extrait d'écorce d'*Acacia catechu*, l'alpha-tocophérol, les anthocyanines, un extrait de feuilles d'*Argania spinosa,* un extrait d'*Aronia* (aronia), l'acide ascorbique et ses esters, un extrait d'*Aspalathus linearis* (rooibos), la baïcaline, le bakuchiol, un extrait de bambou, un extrait de feuilles de bambou, la benfotiamine, l'hydroxytoluène butylé (BHT), un extrait de *Camellia sinensis,* les catéchines, les acides chlorogéniques, un extrait de *Chondrus crispus* (carraghénane), un extrait d'écorces d'agrumes, un extrait de cacao, les polyphénols de cacao, un extrait de canneberge, la décarboxycarnosine HCl, le syringylidène malonate de diéthylhexyle, le gallate d'épigallicathécine (EGCG), le chlorure d'éthylbisiminométhylguaiacol manganèse (EUK-134), l'acide férulique, le monophosphate d'acide férulique, un extrait de *Ficus carica* (figue commune), un extrait de pépins de raisin, un extrait de peau de raisin, un extrait de 4-hydroxy-isoleucine, l'acide 6-hydroxy-2,5,7,8-tétraméthylchromane-2-carboxylique et son phosphate, le malonate d'hydroxydiméthoxybenzyle, un extrait d'*Hypoxis hemerocallidea* (pomme de terre africaine), l'idébénone, le kaempférol, un extrait de graines de kiwi, la poudre de noix de kola (cola), la L-ergothionéine, la L-histidine, l'acide lipoïque et ses esters, un extrait de graines de litchi, la L-2-oxo-thiazolidine (OTZ), la lutéine, la lutéoline, le gluconate de manganèse, le mannitol, un extrait de feuilles de *Morinda citrifolia* (noni), la myricétine, la N-acétyl-cystéine (NAC), le pentagalloylglucose (PGG), un extrait de *Phyllantus emblica* (groseille à maquereau indienne), un extrait de *Punica granatum* (grenade), la quercétine, la quercitrine, un extrait de vin rouge, le resvératrol et ses dérivés, un extrait de *Ribes nigrum* (cassis), un extrait de *Rhodiola rosea* (racine d'or), un extrait de fruit de *Rosa roxburghii* (Chesnut rose), un extrait de romarin, l'acide rosmarinique, la rutine, l'acide salicylique, le phosphate cyclique de salicyloyle, un extrait de *Scutellaria,* un extrait de racines de *Scutellaria baicaleszsis,* un extrait de *Siringa vulgaris* (lilas), un extrait d'épinard, les tétrahydrocurcuminoïdes, le tocophérol et ses esters, un extrait de vanille, les polyphénols du noyer, de préférence le gamma-oryzanol, et leurs mélanges ou leurs combinaisons.

11. Composition à usage topique selon l'une quelconque des revendications 7 à 10, dans laquelle le second antioxydant est choisi dans le groupe constitué par le palmitate d'ascorbyle, le stérate d'ascorbyle, l'hydroxyanisole butylé (BHA), l'hydroxytoluène butylé (BHT), un extrait de *Caesalpinia paraensis,* les acides chlorogéniques, un extrait de *Polypodium leucotomos*, un extrait d'écorce d'*Acacia catechu,* l'alpha-tocophérol, les anthocyanines, un extrait de feuilles d'*Argania spinosa,* un extrait d'*Aronia* (aronia), l'acide ascorbique et ses esters, le maléate d'ascorbyltocophéryle (2-CME), un extrait d'*Aspalathus linearis* (rooibos), la baïcaline, le bakuchiol, un extrait de bambou, un extrait de feuilles de bambou, la benfotiamine, l'hydroxytoluène butylé (BHT), un extrait de *Camellia sinensis,* l'huile de fleurs de *Chamomilla recutita* (matricaire), les catéchines, les acides chlorogéniques, un extrait de *Chondrus crispus* (carraghénane), un extrait d'écorces d'agrumes, un extrait de cacao, les polyphénols de cacao, un extrait de canneberge, la décarboxycarnosine HCl, le syringylidène malonate de diéthylhexyle, le gallate d'épigallicathécine (EGCG), le chlorure d'éthylbis-iminométhylguaiacol manganèse, l'acide férulique, le monophosphate d'acide férulique, un extrait de *Ficus carica* (figue commune), le gamma-oryzanol, un extrait de peau de *Garcinia mangostana*, la glucosylrutine, un extrait de pépins de raisin, un extrait de peau de raisin, un extrait de 4-hydroxy-isoleucine, l'acide 6-hydroxy-2,5,7,8-tétraméthylchromane-2-carboxylique et son phosphate, le malonate d'hydroxydiméthoxybenzyle, un extrait d'*Hypoxis hemerocallidea* (pomme de terre africaine), l'idébénone, le kaempférol, un extrait de graines de kiwi, la poudre de noix de kola (cola), la L-ergothionéine, la L-histidine, un extrait de graines d'*Helianthus annuus* (tournesol), un extrait de réglisse, l'acide lipoïque et ses esters, un extrait de graines de litchi, la L-2-oxo-thiazolidine (OTZ), la lutéine, la lutéoline, le gluconate de manganèse, le mannitol, un extrait d'écorce de *Mimosa tenuiflora,* un extrait de feuilles de *Morinda citrifolia* (noni), l'acide nordihydroguaiarétique (NDGA), la myricétine, la N-acétyl-cystéine (NAC), un extrait de fleur de *Nymphea alba* (nénuphar), un extrait de graines d'*Oenothera biennis* (onagre), un extrait d'*Oryza sativa* (riz), le pentagalloylglucose (PGG), un extrait de graines de *Perilla ocymoides,* un extrait de *Phyllantus emblica* (groseille à maquereau indienne), un extrait de fruit de *Pimpinella anisum* (anis vert), un extrait d'écorces de *Pinus pinaster* (pin maritime français), un extrait de graines de *Psoralea corylifolia,* un extrait de *Punica granatum* (grenade), la quercétine, l'hydrate de quercétine, la quercitrine, un extrait de vin rouge, le resvératrol et ses dérivés, un extrait de *Ribes nigrum* (cassis), un extrait de *Rhodiola rosea* (racine d'or), un extrait de fruit de *Rosa roxburghii* (Chesnut rose), un extrait de romarin, l'acide rosmarinique, la rutine, l'hydrate de rutine, l'acide salicylique, le phosphate cyclique de salicyloyle, l'azoture de sodium, un extrait de *Scutellaria,* un extrait de racines de *Scutellaria baicaleszsis,* un extrait de *Siringa vulgaris* (lilas), un extrait d'épinard, les tétrahydro-curcuminoïdes, un ferment de *Thermus thermophillus,* le tocophérol et ses esters, les tocotriénols, l'ubiquinone, un extrait de *Vaccinium myrtillis* (myrtille), un extrait de vanille, les polyphénols du noyer, un extrait de wasabi, une xanthophylle, de préférence le maléate d'ascorbyltocophéryle, et leurs mélanges ou leurs combinaisons.

12. Composition à usage topique selon l'une quelconque des revendications 7 à 11, comprenant en outre au moins un composé organique sensible à la décomposition ou à la dégradation oxydative, ledit composé organique étant de préférence choisi parmi un colorant organique ou un agent écran solaire organique.
